# EUROPÄISCHE PATENTSCHRIFT

(11) **EP 1 986 983 B1**
(45) Veröffentlichungstag und Bekanntmachung des Hinweises auf die Patenterteilung: **22.07.2009**
(21) Anmeldenummer: 07764516.6
(22) Anmeldetag: 25.01.2007
(51) Int. Cl.: C07B 41/06, C07C 45/59

(54) **SPALTUNG VON DIALKOXYALKANEN IN IONISCHEN FLÜSSIGKEITEN**
SPLITTING OF DIALKOXYALKANES IN IONIC LIQUIDS
DÉCOMPOSITION DE DIALCOXYALCANES DANS DES LIQUIDES IONIQUES

(30) Priorität: 23.02.2006 DE 102006008424
(43) Veröffentlichungstag der Anmeldung: 05.11.2008
(73) Patentinhaber: Merck Patent GmbH, 64293 Darmstadt (DE)
(72) Erfinder: IGNATYEV, Nikolai (Mykola), 47058 Duisburg (DE); KOPPE, Karsten, 45768 Marl (DE); FROHN, Hermann, Josef, 47906 Kempen (DE); BARTHEN, Peter, 47495 Rheinberg (DE)
(86) Internationale Anmeldenummer: PCT/EP2007/000633
(87) Internationale Veröffentlichungsnummer: WO 2007/104380

(56) Entgegenhaltungen:
- US-A- 4 161 614

## Beschreibung

Die vorliegende Erfindung betrifft ein Verfahren zur Spaltung von Dialkoxyalkanen unter Erhalt entsprechender Aldehyde oder Ketone, wobei die Spaltung in Anwesenheit mindestens einer lonischen Flüssigkeit der allgemeinen Formel K⁺A⁻ erfolgt.

Carbonylgruppen sind regelmäßiger Bestandteil von organischen Verbindungen, z.B. von Pharrnawirkstoffen, Pflanzenschutzmitteln, Polymeren oder Vorstufen in der Feinchemie. Im Rahmen der Synthese ist es vielfach erforderlich, die Carbonylgruppen durch eine entsprechende Schutzgruppe zu schützen, um weitere Modifikationen an der jeweiligen Verbindung vornehmen zu können, z.B. durch Umsetzung mit metallorganischen Reagenzien, die mit der Carbonylgruppe in einer Nebenreaktion reagieren würden.

Als gängige Schutzgruppe für Carbonylfunktionen von Aldehyden und Ketonen haben sich Ketale bzw. Acetale etabliert. Hierbei handelt es sich um Dialkoxyalkane, die aus den entsprechenden Carbonylverbindungen durch Umsetzung mit Alkoholen erhalten werden. Diese geminalen Diether sind unreaktiv gegenüber einer Reihe von Reagenzien, z.B. metallorganischen Reagenzien. Bei Bedarf können die Carbonylgruppen durch Spaltung der Ketale oder Acetale unter Freisetzung entsprechender Alkohole wieder erhalten werden. Üblicherweise erfolgt die Spaltung der Dialkoxyalkane durch Umsetzung mit Säuren, wie beispielsweise in A. Streitwieser, C.H. Heathcock, E.M. Kosover, Organische Chemie, 2. Auflage, VCH, Weinheim 1994, 402-403 beschrieben. Hierzu werden üblicherweise Mineralsäuren oder starke organische Säuren, wie zum Beispiel p-Toluolsulfonsäure, eingesetzt. Diese Umsetzung erfolgt in organischen Lösungsmitteln, die nach der Reaktion zusammen mit den Nebenprodukten von der gewünschten Carbonylverbindung abgetrennt werden müssen. Die Verwendung von organischen Lösungsmitteln und starker Säuren erweist sich aber, insbesondere in großtechnischen Prozessen, als nachteilig, weil große Mengen an Lösungsmitteln, die zum Teil mit starken Säuren versetzt sind, entsorgt werden müssen. Gerade für großtechnische Synthesen erweisen sich damit die genannten Methoden als nachteilig, da insbesondere bei Einsatz von Lösungsmitteln zusätzliche sicherheitstechnische Aspekte zur Vermeidung von Belastungen der Umwelt und zum Brandschutz berücksichtigt werden müssen.

Es besteht damit ein Bedarf an Alternativen zur Spaltung von Dialkoxyalkanen unter Freisetzung der entsprechenden Aldehyde oder Ketone, die einfach durchzuführen sind, eine hohe Ausbeute der entsprechenden Aldehyde und Ketone liefern, und dabei die Verwendung großer Mengen von Lösungsmitteln vermeiden.

Demgemäß ist die Aufgabe der vorliegenden Erfindung, ein Verfahren bereit zu stellen, das die oben genannten Anforderungen erfüllt, dass gut beherrschbar ist, keinen hohen sicherheitstechnischen Aufwand erfordert und damit auch eine ökonomische Spaltung von Ketalen oder Acetalen in kommerziellen Maßstäben erlaubt. Überraschenderweise wurde gefunden, dass lonische Flüssigkeiten in idealer Weise als alternatives Lösungsmittel für die Spaltung von Dialkoxyalkanen geeignet sind.

Gegenstand der vorliegenden Erfindung ist demgemäß ein Verfahren zur Spaltung von Dialkoxyalkanen der allgemeinen Formel (I) unter Erhalt von Aldehyden oder Ketonen 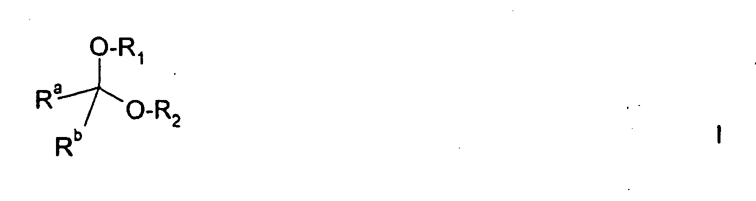 wobei
R^{a} und R^{b} unabhängig voneinander einen ggf. substituierten aliphatischen oder aromatischen Rest, der ein oder mehrere Heteroatome aufweisen kann, bedeuten, wobei einer der Reste R^{a} oder R^{b} auch H bedeuten kann, und wobei die beiden Reste R^{a} und R^{b} miteinander verbunden sein können, und wobei
R₁ und R₂ unabhängig voneinander einen ggf. substituierten gesättigten oder ungesättigten aliphatischen oder aromatischen Rest, der ein oder mehrere Heteroatome aufweisen kann, bedeuten und wobei die beiden Reste R₁ und R₂ miteinander verbunden sein können, **dadurch gekennzeichnet, dass** die Spaltung in Anwesenheit mindestens einer lonischen Flüssigkeit der allgemeinen Formel K⁺A⁻ erfolgt. Es hat sich herausgestellt, dass lonische Flüssigkeiten zur Durchführung der Spaltung von Dialkoxyalkanen in besonderer Weise geeignet sind. Bei der Durchführung des erfindungsgemäßen Verfahrens kann die mindestens eine lonische Flüssigkeit in einer Mischung mit weiteren Lösungsmitteln, insbesondere organischen Lösungsmitteln vorliegen, vorzugsweise erfolgt das Verfahren ohne Zusatz eines weiteren Lösungsmittels, d.h. allein in Gegenwart mindestens einer Ionischen Flüssigkeit.

lonische Flüssigkeiten oder flüssige Salze sind ionische Spezies, die aus einem organischen Kation (K⁺) und einem in der Regel anorganischen Anion (A⁻) bestehen. Sie enthalten keine neutralen Moleküle und weisen meistens Schmelzpunkte kleiner 373 K auf.

Das Gebiet der ionischen Flüssigkeiten wird zurzeit intensiv erforscht, da die Anwendungsmöglichkeiten vielfältig sind. Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "lonic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton ,,Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Da es sich bei lonischen Flüssigkeiten um Salze handelt, weisen sie keine Flüchtigkeit auf und setzen damit auch keine entzündlichen oder giftigen Dämpfe frei. Sie stellen damit ein sicheres Medium zur Durchführung der Ketalspaltung dar. Darüber hinaus hat sich herausgestellt, dass bei Einsatz von lonischen Flüssigkeiten zur Spaltung von Dialkoxyalkanen der sonst übliche Zusatz von Säure zur Katalysierung der Reaktion nicht unbedingt erforderlich ist. Die lonische Flüssigkeit selbst kann damit die gewünschte Wiederfreisetzung der Carbonylfunktion katalysieren. Im einfachsten Fall kann das erhaltene Aldehyd oder Keton von der lonischen Flüssigkeit dekantiert werden und ohne weitere Aufreinigung weiter eingesetzt werden. Auch die lonische Flüssigkeit lässt sich auf diese Weise einfach recyceln und kann mehrfach wieder verwendet werden. Insgesamt ergibt sich mit dem erfindungsgemäßen Verfahren ein einfacher und kostengünstiger Prozess, der auch für die Synthese im kommerziellen Maßstab geeignet ist.

### Geeignete Dialkoxyalkane sind jene gemäß der Formel (I)

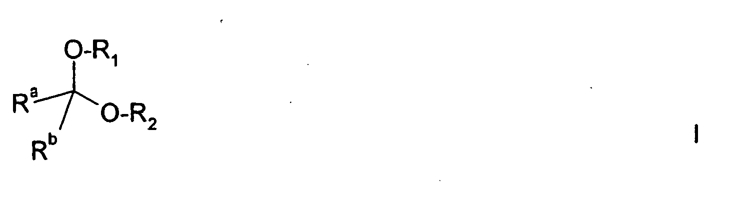

wobei
R^{a} und R^{b} unabhängig voneinander einen ggf. substituierten aliphatischen Rest oder aromatischen Rest, der ein oder mehrere Heteroatome aufweisen kann, bedeuten, wobei einer der Reste R^{a} oder R^{b} auch H bedeuten kann, und wobei die beiden Reste R^{a} und R^{b} miteinander verbunden sein können, und wobei
R₁ und R₂ unabhängig voneinander einen ggf. substituierten gesättigten oder ungesättigten aliphatischen oder aromatischen Rest, der ein oder mehrere Heteroatome aufweisen kann, bedeuten und wobei die beiden Reste R₁ und R₂ miteinander verbunden sein können.

R₁ und R₂ können unabhängig voneinander gesättigte oder ungesättigte aliphatische Reste oder aromatische Reste sein. Geeignete aliphatische Reste sind vorzugsweise geradkettige oder verzweigte Alkylgruppen mit 1 bis 10 C-Atomen, insbesondere sind R₁ und R₂ jeweils unabhängig voneinander Methyl, Ethyl, n-Propyl, Isopropyl, n-Butyl, tert.-Butyl, sek.-Butyl, Phenyl oder Cyclohexyl, ganz besonders bevorzugt Methyl, Ethyl, n-Propyl, Isopropyl oder n-Butyl. Geeignete aromatische Reste sind beispielsweise Phenyl, Benzyl.

Vorzugsweise sind die Reste R^{a} und R^{b} sowie R₁ und R₂ miteinander verbunden, beispielsweise unter Ausbildung eines aliphatischen und/oder aromatischen Rings oder anellierten Ringsystems, der bzw. das auch ein oder mehrere Heteroatome aufweisen kann.

Ganz besonders bevorzugt sind R₁ und R₂ unter Ausbildung eines aliphatischen gesättigten oder ungesättigten Rings miteinander verbunden, das heißt R₁ und R₂ bilden zusammen eine gesättigte oder ungesättigte Alkylkette , wobei die Alkylkette 1 bis 10 C-Atome haben kann. Vorzugsweise hat die Alkylkette 2 bis 5 C-Atome, so dass insgesamt ein Dialkoxyalkan mit einem 5- bis 8-gliedrigen Ring vorliegt. Ganz besonders bevorzugt weist die Alkylkette 2 C-Atome auf, so dass ein Dialkoxyalkan mit einem 5-gliedrigen Ring vorliegt. Diese besonders bevorzugten Dialkoxyalkane werden durch Umsetzung der entsprechenden Carbonylverbindung mit 1,2-Ethandiol erhalten. Die Bildung eines Fünfrings ist dabei besonders begünstigt und daher zu bevorzugen.

Vorzugsweise besitzt R^{a} eine Bedeutung gemäß Formel la
worin
- R^{e}: einen unsubstituierten, einen einfach oder mehrfach durch CN und/oder Halogen substituierten Alkylrest mit 1 bis 15 C-Atomen, wobei in diesen Resten auch eine oder mehrere CH₂-Gruppen durch -O-, -S-, -CH=CH-, -C≡C-, -OC-O- und/oder -O-CO-, und/oder auch eine oder mehrere CH-Gruppen durch N oder P so ersetzt sein können, dass zwei O-Atome nicht direkt miteinander verknüpft sind, oder, falls r und/oder p verschieden von 0 sind, auch H, Halogen, CN, SF₅ oder NCS,
- A⁰, A¹: jeweils unabhängig voneinander
a) einen 1,4-Cyclohexenylen- oder 1,4-Cyclohexylenrest, worin eine oder zwei nicht benachbarte CH₂-Gruppen durch -O-oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl, 1,4-Bicyclo[2,2,2]octylen, Phenanthren-2,7-diyl, Naphthalin-2,6-diyl, Decahydronaphthalin-2,6-diyl, und 1,2,3,4-Tetrahydronaphthalin-2,6-diyl,
d) einen bivalenten Rest aus der Gruppe Furan, Pyrrol, Thiophen, Pyrazol, Imidazol, 1,2-Oxazol, 1,3-Oxazol, Thiazol, Pyridin, Pyridazin, Pyrimidin, Pyrazin, 2H-Pyran, 4H-Pyran, Purin, Pteridin, 1 H-Azepin, 3H-1,4-Diazepin, Indol, Benzofuran, Benzothiophen, Chinolin, Isochinolin, Phenazin, Phenoxazin, Phenothiazin und 1,4-Benzodiazepin, 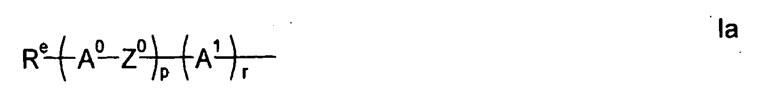
wobei die Reste a), b), c) und d) ein- oder mehrfach durch R^{e}, insbesondere durch Halogen und/oder CN, substituiert sein können,
- Z⁰: -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- oder eine Einfachbindung,
- p: 0, 1, 2 oder 3 und
- r: 0, 1 oder 2 bedeutet.

Bevorzugte Bedeutungen von R^{e} sind geradkettige oder verzweigte Alkyl-und Alkoxyreste mit 1 bis 8 C-Atomen, die einfach durch -CN und/oder ein-oder mehrfach durch Halogen substituiert sein können.

Bevorzugte Bedeutungen von A⁰ und/oder A¹ sind 1,4-Cyclohexylen, worin eine oder zwei nicht benachbarte CH₂-Gruppen durch -O- ersetzt sein können, 1,4-Phenylen, worin eine oder zwei CH-Gruppen durch N ersetzt sein können, Phenanthren-2,7-diyl-, Naphthalin-2,6-diyl, Decahydronaphthalin-2,6-diyl, und 1,2,3,4-Tetrahydronaphthalin-2,6-diyl, wobei diese Reste ein- oder mehrfach durch Halogen, insbesondere Fluor und/oder Chlor, CN und/oder gegebenenfalls durch Halogen substituiertes C₁₋₅-Alkyl oder -Alkoxy substituiert sein können.

R^{a} kann auch Bestandteil eines Liganden, beispielsweise eines Cyclopentadienyl-Systems in einem metallorganischen Komplex, sein.

### Besonders bevorzugte Gruppen R^{a} sind nachfolgend aufgeführt:

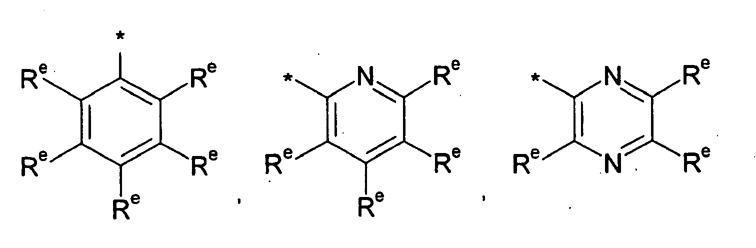

und 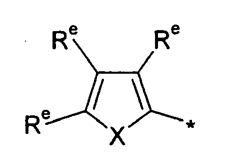 worin X O, NR^{e} oder S bedeutet, R^{e} die angegebene Bedeutung besitzt und * die freie Bindung angibt.

Vor- und nachstehend bedeutet Halogen als Substituent organischer Reste Fluor, Chlor, Brom oder Iod, vorzugsweise Fluor oder Chlor, besonders bevorzugt Fluor.

Vor- und nachstehend können mehrfach vorkommende Gruppen und Substituenten, wie beispielsweise A⁰, Z⁰, A¹, R^{e}, jeweils gleiche oder unterschiedliche Bedeutungen aufweisen.

Vorzugsweise besitzt der Rest R^{a} eine Bedeutung gemäß der Formel la und der übrige Rest R^{b} bedeutet H.

Weiterhin bevorzugt sind R^{a} und R^{b} derart miteinander verbunden, dass die Dialkoxyverbindung der Formel I eine Bedeutung gemäß Formel Ib besitzt 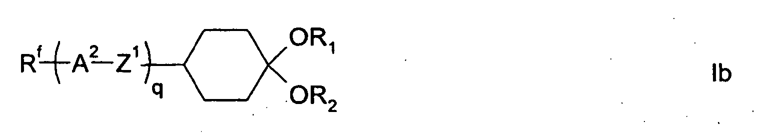 worin
- R^{f}: eine der zu R^{e} angegebenen Bedeutungen besitzt,
- A²: eine der zu A⁰, A¹ angegebenen Bedeutungen besitzt,
- Z¹: eine der zu Z⁰ angegebenen Bedeutungen besitzt,
- q: 0, 1, 2 oder 3 bedeutet und
- R₁ und R₂: die zuvor und nachfolgend angegebenen Bedeutungen besitzen.

Wesentlich für das erfindungsgemäße Verfahren zur Spaltung Dialkoxysilanen sind die lonischen Flüssigkeiten der allgemeinen Formel K⁺A⁻. Dabei spielt die Auswahl des Anions A⁻ der lonischen Flüssigkeit eine besondere Rolle. Vorzugsweise handelt es sich bei dem Anion A⁻ um ein Anion einer korrespondierenden starken Säure. Insbesondere ist das Anion A⁻ der lonischen Flüssigkeit ausgewählt aus der Gruppe [HSO₄]⁻, [SO₄]²⁻, [NO₃]⁻, [BF₄]⁻, [(R_{F})BF₃]⁻, ((R_{F})₂BF₂]⁻, [(R_{F})₃BF]⁻, [(R_{F})₄B]⁻, [B(CN)₄]⁻, [PO₄]³⁻, [HPOₐ]²⁻, [H₂PO₄]⁻, [Alkyl-OPO₃]²⁻, [(Alkyl-O)₂PO₂]⁻, [Alkyl-PO₃]²⁻, [R_{F}PO₃]²⁻, [(Alkyl)₂PO₂]⁻, [(R_{F})₂PO₂]⁻, [R_{F}SO₃]⁻, [HOSO₂(CF₂)ₙSO₂O]⁻, [OSO₂(CF₂)ₙSO₂O]²⁻, [Alkyl-SO₃]⁻, [HOSO₂(CH₂)ₙSO₂O]⁻, [OSO₂(CH₂)ₙSO₂O]²⁻, [Alkyl-OSO₃]⁻, [Alkyl-C(O)O]-, [HO(O)C(CH₂)ₙC(O)O]⁻, [R_{F}C(O)O]⁻, [HO(O)C(CF₂)ₙC(O)O]⁻, [O(O)C(CF₂)ₙC(O)O]²⁻, [(R_{F}SO₂)₂N]⁻, [(FS0₂)₂N]⁻, [((R_{F})₂P(O))₂N]⁻, [(R_{F}SO₂)₃C]⁻, [(FSO₂)₃C]⁻, Cl⁻ und/oder Br⁻
wobei
n = 1 bis 8 bedeutet
R_{F} die Bedeutung fluoriertes Alkyl

(CₘF₂ₘ₋ₓ₊₁Hₓ)

mit m = 1-12 und x = 0- 7 hat, wobei für m = 1 x = 0 bis 2 sein soll, und/oder fluoriertes (auch perfluoriertes) Aryl oder Alkyl-Aryl.

Die Alkylgruppe in den oben genannten Anionen kann ausgewählt sein aus geradkettigen oder verzweigten Alkylgruppen mit 1 bis 20 C-Atomen, vorzugsweise mit 1 bis 14 C-Atomen und insbesondere bevorzugt mit 1 bis 4 C-Atomen.

Vorzugsweise bedeutet R_{F} CF₃, C₂F₅, C₃F₇ oder C₄F₉.

In Bezug auf die Wahl des Kations K⁺ der lonischen Flüssigkeit der ionischen Flüssigkeit gibt es per se keine Einschränkungen. Vorzugsweise handelt es sich aber um organische Kationen, wobei es sich insbesondere bevorzugt um Ammonium-, Phosphonium, Thiouronium-, Guanidiniumkationen oder um heterocyclische Kationen handelt.

Ammoniumkationen können beispielsweise durch die Formel (1)

[NR₄]⁺ (1),

beschrieben werden, wobei
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren nicht konjugierten Doppelbindungen,
geradkettiges oder verzweigtes Alkinyl mit 2-20 C-Atomen und einer oder mehreren nicht konjugierten Dreifachbindungen,
gesättigter, teilweise oder vollständig ungesättigter Cycloaliphat mit 3-7 C-Atomen, das mit Alkylgruppen mit 1-6 C-Atomen substituiert sein kann, bedeutet, wobei ein oder mehrere R teilweise oder vollständig mit Halogenen, insbesondere -F und/oder -Cl, oder teilweise mit -OR', -CN, - C(O)OH, -C(O)NR'₂, -SO₂NR'₂, -SO₂OH, -SO₂X, -NO₂, substituiert sein können, und wobei ein oder zwei nicht benachbarte und nicht α-ständige Kohlenstoffatome des R, durch Atome und/oder Atomgruppierungen ausgewählt aus der Gruppe -O-, -S-, -S(O)-, -SO₂-, -N⁺R'₂-, -C(O)NR'-, - S0₂NR'-, oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen sein kann.

Phosphoniumkationen können beispielsweise durch die Formel (2)

[PR²₄]⁺ (2),

beschrieben werden, wobei
R² jeweils unabhängig voneinander
H, NR'₂
geradkettiges oder verzweigtes Alkyl mit 1-20 C-Atomen,
geradkettiges oder verzweigtes Alkenyl mit 2-20 C-Atomen und einer oder mehreren nicht konjugierten Doppelbindungen,
geradkettiges oder verzweigtes Alkinyl mit 2-20 C-Atomen und einer oder mehreren nicht konjugierten Dreifachbindungen,
gesättigter, teilweise oder vollständig ungesättigter Cycloaliphat mit 3-7 C-Atomen, das mit Alkylgruppen mit 1-6 C-Atomen substituiert sein kann, bedeutet, wobei ein oder mehrere R² teilweise oder vollständig mit Halogenen, insbesondere -F und/oder -Cl, oder teilweise mit -OR', -CN, - C(O)OH, -C(O)NR'₂, -SO₂NR'₂, -SO₂OH, -SO₂X, -NO₂, substituiert sein können, und wobei ein oder zwei nicht benachbarte und nicht α-ständige Kohlenstoffatome des R², durch Atome und/oder Atomgruppierungen ausgewählt aus der Gruppe -O-, -S-, -S(O)-, -SO₂-, -N⁺R'₂-, -C(O)NR'-, - SO₂NR'-, oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen.

Ausgeschlossen sind jedoch Kationen der Formeln (1) und (2), in denen alle vier oder drei Substituenten R und R² vollständig mit Halogenen substituiert sind, beispielsweise das Tris(trifluormethyl)methylammonium-kation, das Tetra(trifluormethyl)ammoniumkation oder das Tetra(nonafluorbutyl)ammoniumkation.

Geeignete Thiouroniumkationen können durch die Formel (3),

[(R³R⁴N)-C(=SR⁵)(NR⁶R⁷)]⁺ (3),

beschrieben werden, wobei
R³ bis R⁷ jeweils unabhängig voneinander
Wasserstoff,
geradkettiges oder verzweigtes Alkyl mit 1 bis 20 C-Atomen,
geradkettiges oder verzweigtes Alkenyl mit 2-20 C-Atomen und einer oder mehreren nicht konjugierten Doppelbindungen,
geradkettiges oder verzweigtes Alkinyl mit 2-20 C-Atomen und einer oder mehreren nicht konjugierten Dreifachbindungen,
gesättigter, teilweise oder vollständig ungesättigter Cycloaliphat mit 3-7 C-Atomen, das mit Alkylgruppen mit 1-6 C-Atomen substituiert sein kann, bedeutet, wobei ein oder mehrere der Substituenten R³ bis R⁷ teilweise oder vollständig mit Halogenen, insbesondere -F und/oder -CI, oder teilweise mit -OH, -OR', -CN, -C(O)OH, -C(O)NR'₂, -SO₂NR'₂, -SO₂OH, - SO₂X, -NO₂, substituiert sein können, und wobei ein oder zwei nicht benachbarte und nicht α-ständige Kohlenstoffatome von R³ bis R⁷ durch Atome und/oder Atomgruppierungen ausgewählt aus der Gruppe -O-, -S-, - S(O)-, -SO₂-, -N⁺R'₂-, -C(O)NR'-, -SO₂NR'-, oder -P(O)R'- ersetzt sein können mit R' = H, nicht, teilweise oder perfluoriertes C₁- bis C₆-Alkyl, C₃-bis C₇-Cycloalkyl, unsubstituiertes oder substituiertes Phenyl und X =Halogen.

Guanidiniumkationen können durch die Formel (4)

[C(NR⁸R⁹)(NR¹⁰R¹¹)(NR¹²R¹³)]⁺ (4),

beschrieben werden, wobei
R⁸ bis R¹³ jeweils unabhängig voneinander
Wasserstoff, -CN, NR'₂,
geradkettiges oder verzweigtes Alkyl mit 1 bis 20 C-Atomen,
geradkettiges oder verzweigtes Alkenyl mit 2-20 C-Atomen und einer oder mehreren nicht konjugierten Doppelbindungen,
geradkettiges oder verzweigtes Alkinyl mit 2-20 C-Atomen und einer oder mehreren nicht konjugierten Dreifachbindungen,
gesättigter, teilweise oder vollständig ungesättigter Cycloaliphat mit 3-7 C-Atomen, das mit Alkylgruppen mit 1-6 C-Atomen substituiert sein kann, bedeutet, wobei ein oder mehrere der Substituenten R⁸ bis R¹³ teilweise oder vollständig mit Halogenen, insbesondere -F und/oder -Cl, oder teilweise mit -OR', -CN, -C(O)OH, -C(O)NR'₂, -SO₂NR'₂, -SO₂OH, -SO₂X, - NO₂, substituiert sein können, und wobei ein oder zwei nicht benachbarte und nicht α-ständige Kohlenstoffatome von R⁸ bis R¹³ durch Atome und/oder Atomgruppierungen ausgewählt aus der Gruppe -O-, -S-, -S(O)-, - SO₂-, -N⁺R'₂-, -C(O)NR'-, -SO₂NR'-, oder -P(O)R'- ersetzt sein können mit
R' = H, nicht, teilweise oder perfluoriertes C₁- bis C₆-Alkyl, C₃- bis C₇-Cycloalkyl, unsubstituiertes oder substituiertes Phenyl und X = Halogen.

Darüber hinaus können Kationen der allgemeinen Formel (5)

[HetN]⁺ (5)

eingesetzt werden, wobei
HetN⁺ ein heterocyclisches Kation, ausgewählt aus der Gruppe 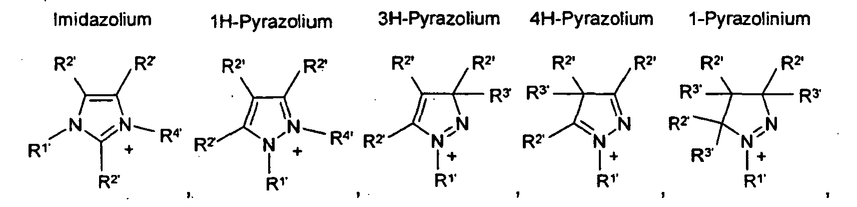 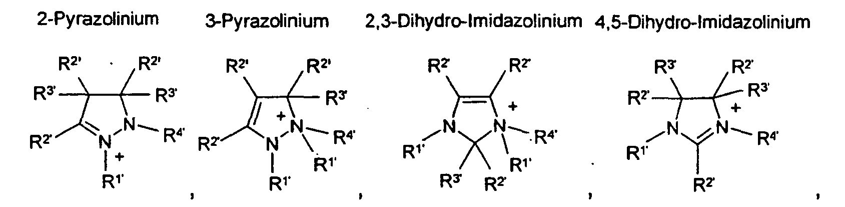 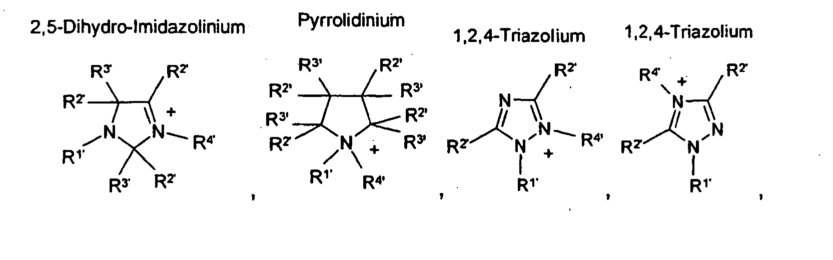 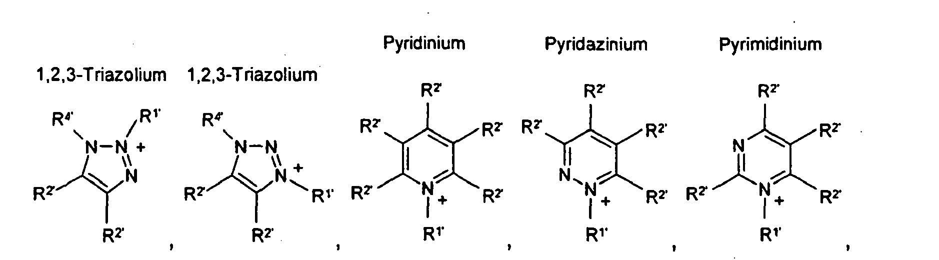 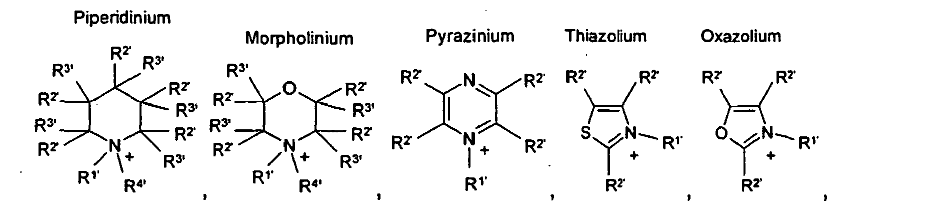 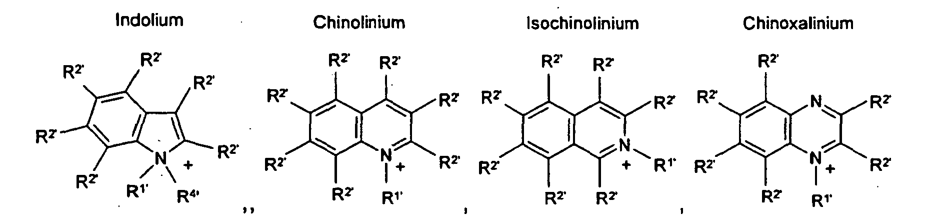 oder 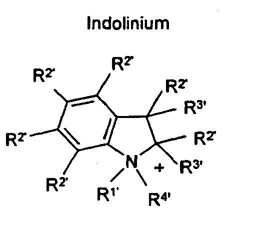 bedeutet, wobei die Substituenten
R¹' bis R⁴' jeweils unabhängig voneinander
Wasserstoff, -CN,
geradkettiges oder verzweigtes Alkyl mit 1-20 C-Atomen,
geradkettiges oder verzweigtes Alkenyl mit 2-20 C-Atomen und einer oder mehreren nicht konjugierten Doppelbindungen,
geradkettiges oder verzweigtes Alkinyl mit 2-20 C-Atomen und einer oder mehreren nicht konjugierten Dreifachbindungen,
gesättigter, teilweise oder vollständig ungesättigter Cycloaliphat mit 3-7 C-Atomen, das mit Alkylgruppen mit 1-6 C-Atomen substituiert sein kann, gesättigtes, teilweise oder vollständig ungesättigtes Heteroaryl,
Heteroaryl-C₁-C₆-alkyl oder Aryl-C₁-C₆-alkyl bedeutet,
wobei die Substituenten R^{1'}, R^{2'}, R^{3'} und/oder R^{4'} zusammen auch ein Ringsystem bilden können,
wobei ein oder mehrere Substituenten R¹' bis R⁴' teilweise oder vollständig mit Halogenen, insbesondere -F und/oder -Cl, oder -OR', -CN, -C(O)OH, - C(O)NR'₂, -SO₂NR'₂, -C(O)X, -SO₂OH, -SO₂X, -NO₂, substituiert sein können, wobei jedoch nicht gleichzeitig R^{1'} und R^{4'} vollständig mit Halogenen substituiert sein dürfen, und wobei ein oder zwei nicht benachbarte und nicht am Heteroatom gebundene Kohlenstoffatome der Substituenten R¹' bis R⁴', durch Atome und/oder Atomgruppierungen ausgewählt aus der -O-, -S-, -S(O)-, -SO₂-, -N⁺R'₂-, -C(O)NR'-, -SO₂NR'-, oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen.

Als Substituent R^{2'} eignen sich insbesondere auch Atomgruppierungen ausgewählt aus -OR',-NR'₂, -C(O)OH, -C(O)NR'₂, -SO₂NR'₂, -SO₂OH, - SO₂X oder -NO₂.

Unter vollständig ungesättigten Substituenten werden im Sinne der vorliegenden Erfindung auch aromatische Substituenten verstanden.

Als Substituenten R und R² bis R¹³ der Verbindungen der Formeln (1) bis (4) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloaliphatgruppen, die mit C₁- bis C₆-Alkylgruppen substituiert sein können, insbesondere Phenyl.

Die Substituenten R und R² in den Verbindungen der Formel (1) oder (2) können dabei gleich oder verschieden sein. Bevorzugt sind die Substituenten R und R² verschieden.

Die Substituenten R und R² sind insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations [C(NR⁸R⁹)(NR¹⁰R¹¹)(NR¹²R¹³)]⁺ können auch paarweise derart verbunden sein, dass mono-, bi- oder polycyclische Kationen entstehen. Ohne Einschränkung der Allgemeinheit sind Beispiele für solche Guanidinium-Kationen: 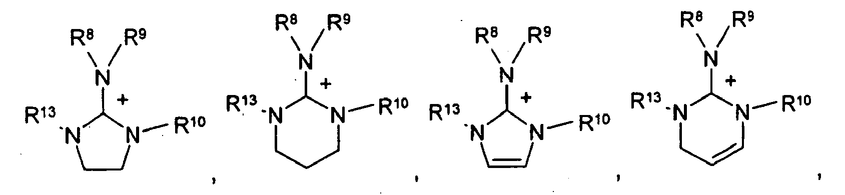 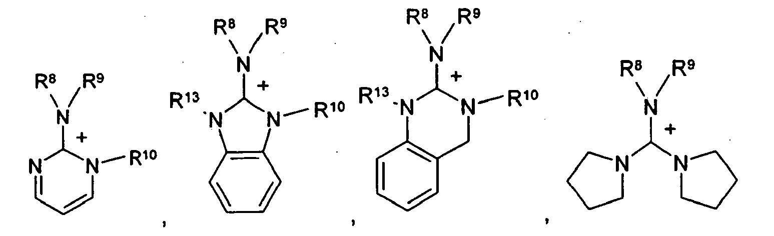 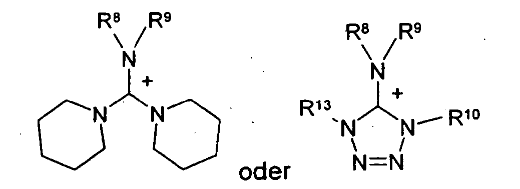 wobei die Substituenten R⁸ bis R¹⁰ und R¹³ eine zuvor angegebene oder besonders bevorzugte Bedeutung haben können. Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, CN, NR'₂, F, Cl, Br, I, C₁-C₆-Alkoxy, SCF₃, SO₂CF₃, COOH, SO₂NR'₂, SO₂X' oder SO₃H substituiert sein, wobei X und R' eine zuvor angegebene Bedeutung haben, substituiertes oder unsubstituiertes Phenyl oder unsubstituierter oder substituierter Heterocyclus substituiert sein.

Bis zu vier Substituenten des Thiouroniumkations [(R³R⁴N)-C(=SR⁵)(NR⁶R⁷)]⁺ können auch paarweise derart verbunden sein, dass mono-, bi- oder polycyclische Kationen entstehen.

Ohne Einschränkung der Allgemeinheit sind Beispiele für solche Kationen im Folgenden angegeben, wobei Y = S bedeutet: 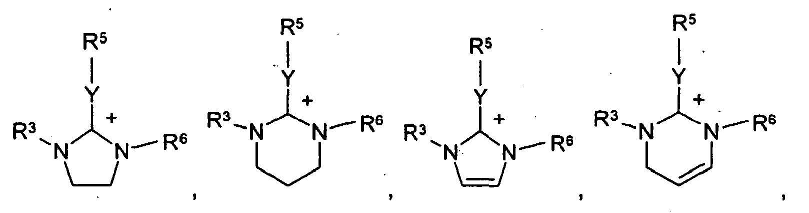 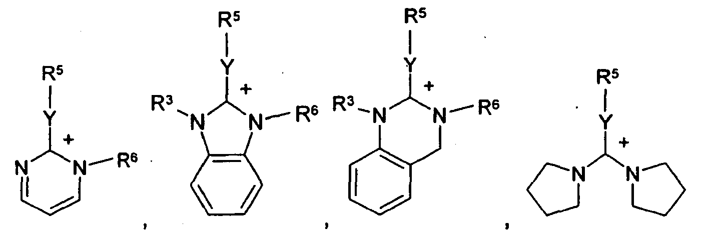 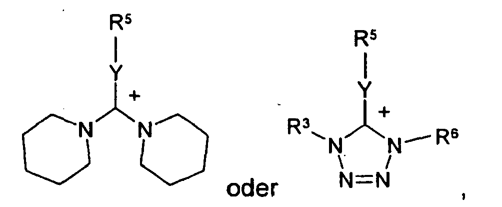 wobei die Substituenten R³, R⁵ und R⁶ eine zuvor angegebene oder besonders bevorzugte Bedeutung haben können. Gegebenenfalls können die Carbocyclen oder Heterocyclen der zuvor angegebenen Kationen noch durch C₁- bis C₆-Alkyl, C₁- bis C₆-Alkenyl, NO₂, CN, NR'₂, F, Cl, Br, I, C₁-C₆-Alkoxy, SCF₃, SO₂CF₃, COOH, SO₂NR'₂, SO₂X oder SO₃H oder substituiertes oder unsubstituiertes Phenyl oder unsubstituierter oder substituierter Heterocyclus substituiert sein, wobei X und R' eine zuvor angegebene Bedeutung haben.

Die Substituenten R³ bis R¹³ sind jeweils unabhängig voneinander bevorzugt eine geradkettige oder verzweigte Alkylgruppe mit 1 bis 10 C-Atomen. Die Substituenten R³ und R⁴, R⁶ und R⁷, R⁸ und R⁹, R¹⁰ und R¹¹ und R¹² und R¹³ in Verbindungen der Formeln (3) bis (4) können dabei gleich oder verschieden sein. Besonders bevorzugt sind R³ bis R¹³ jeweils unabhängig voneinander Methyl, Ethyl, n-Propyl, Isopropyl, n-Butyl, tert.-Butyl, sek.-Butyl, Phenyl oder Cyclohexyl, ganz besonders bevorzugt Methyl, Ethyl, n-Propyl, Isopropyl oder n-Butyl.

Als Substituenten R^{1'} bis R^{4'} von Verbindungen der Formel (5) kommen erfindungsgemäß dabei neben Wasserstoff bevorzugt in Frage: C₁- bis C₂₀, insbesondere C₁- bis C₁₂-Alkylgruppen, und gesättigte oder ungesättigte, d.h. auch aromatische, C₃- bis C₇-Cycloaliphatgruppen, die mit C₁- bis C₆-Alkylgruppen substituiert sein können, insbesondere Phenyl.

Die Substituenten R^{1'} und R^{4'} sind jeweils unabhängig voneinander insbesondere bevorzugt Methyl, Ethyl, Isopropyl, Propyl, Butyl, sek.-Butyl, Pentyl, Hexyl, Octyl, Decyl, Cyclohexyl, Phenyl oder Benzyl. Sie sind ganz besonders bevorzugt Methyl, Ethyl, n-Butyl oder Hexyl. In Pyrrolidinium-, Piperidinium- oder Indolinium-Verbindungen sind die beiden Substituenten R^{1'} und R^{4'} bevorzugt unterschiedlich.

Der Substituent R^{2'} oder R^{3'} ist jeweils unabhängig voneinander insbesondere Wasserstoff, Methyl, Ethyl, Isopropyl, Propyl, Butyl, sek.-Butyl, tert.-Butyl, Cyclohexyl, Phenyl oder Benzyl. Besonders bevorzugt ist R^{2'} Wasserstoff, Methyl, Ethyl, Isopropyl, Propyl, Butyl oder sek.-Butyl. Ganz besonders bevorzugt sind R^{2'} und R^{3'} Wasserstoff.

Die C₁-C₁₂-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl. Gegebenenfalls Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit -OR', - NR'₂, -CN, -C(O)OH, -C(O)NR'₂, -SO₂NR'₂, -C(O)X, -SO₂OH, -SO₂X, -NO₂ substituiert sein können.

Unsubstituierte gesättigte, teilweise oder vollständig ungesättigte Cycloaliphaten mit 3-7 C-Atomen sind daher Cyclopropyl, Cyclobutyl, Cyclopentyl, Cyclohexyl, Cycloheptyl, Cyclopentenyl, Cyclohexenyl, Phenyl, Cycloheptenyl, welche mit C₁- bis C₆-Alkylgruppen substituiert sein können,
wobei wiederum die Cycloaliphatgruppe oder die mit C₁- bis C₆-Alkylgruppen substituierte Cycloaliphatgruppe auch mit Halogenatomen wie F, CI, Br oder I, insbesondere F oder CI oder mit -OR', -CN, -C(O)OH, - C(O)NR'₂, -SO₂NR'₂, -SO₂OH, -SO₂X, -NO₂ substituiert sein kann.

In den Substituenten R, R² bis R¹³ oder R^{1'} bis R^{4'} können auch ein oder zwei nicht benachbarte und nicht α-ständig zum Heteroatom gebundene Kohlenstoffatome, durch Atome und/oder Atomgruppierungen ausgewählt aus der Gruppe -O-, -S-, -S(O)-, -SO₂-, -N⁺R'₂-, -C(O)NR'-, -SO₂NR'-, oder - P(O)R'- ersetzt werden, mit R' = nicht, teilweise oder perfluoriertes C₁- bis C₆-Alkyl, C₃- bis C₇-Cycloalkyl, unsubstituiertes oder substituiertes Phenyl.

Ohne Einschränkung der Allgemeinheit sind Beispiele für derart modifizierte Substituenten R, R² bis R¹³ und R^{1'} bis R^{4'}:
-OCH₃, -OCH(CH₃)₂, -CH₂OCH₃, -CH₂-CH₂-O-CH₃, -C₂H₄OCH(CH₃)₂, - C₂H₄SC₂H₅, -C₂H₄SCH(CH₃)₂, -S(O)CH₃, -SO₂CH₃, -SO₂C₆H₅, -SO₂C₃H₇, - SO₂CH(CH₃) -SO₂CH₂CF₃, -CH₂SO₂CH₃, -O-C₄H₈-O-C₄H₉, -CF₃, -C₂F₅, - C₃F₇, -C₄F₉, -C(CF₃)₃, -CF₂SO₂CF₃, -C₂F₄N(C₂F₅)C₂F₅, -CHF₂, -CH₂CF₃, - C₂F₂H₃, -C₃FH₆, -CH₂C₃F₇, -C(CFH₂)₃, -CH₂C(O)OH, -CH₂C₆H₅ oder P(O)(C₂H₅)₂.

In R' ist C₃- bis C₇-Cycloaliphat beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, CN, NR'₂, F, Cl, Br, I, C₁-C₆-Alkoxy, SCF₃, SO₂CF₃, COOH, SO₂X', SO₂NR''₂ oder SO₃H substituiertes Phenyl, wobei X' F, Cl oder Br und R'' ein nicht, teilweise oder perfluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m-oder p-Isopropylphenyl, o-, m- oder p-tert.-Butylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-lodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

In R^{1'} bis R^{4'} wird als Heteroaryl ein gesättigter oder ungesättigter mono- oder bicyclischer heterocyclischer Rest mit 5 bis 13 Ringgliedern verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁- bis C₆-Alkenyl, NO₂, CN, NR'₂,F, Cl, Br, I, C₁-C₆-Alkoxy, SCF₃, SO₂CF₃, COOH, SO₂X', SO₂NR'₂ oder SO₃H substituiert sein kann, wobei X' und R' eine zuvor angegebene Bedeutung haben.

Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3-oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1 H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4-oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl.

Unter Heteroaryl-C₁-C₆-alkyl wird nun in Analogie zu Aryl-C₁-C₆-alkyl beispielsweise Pyridinyl-methyl, Pyridinyl-ethyl, Pyridinyl-propyl, Pyridinylbutyl, Pyridinyl-pentyl, Pyridinyl-hexyl verstanden, wobei weiterhin die zuvor beschriebenen Heterocyclen in dieser Weise mit der Alkylenkette verknüpft werden können.

### HetN⁺ ist bevorzugt

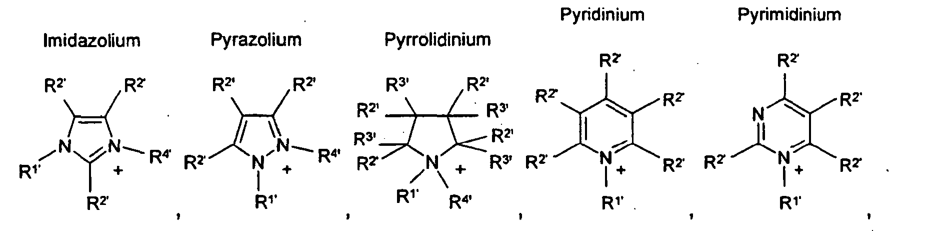

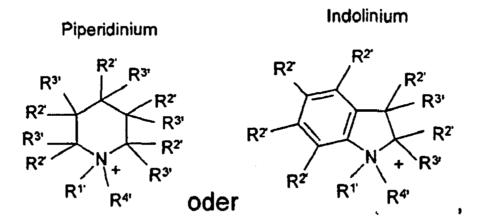

wobei die Substituenten R^{1'} bis R^{4'} jeweils unabhängig voneinander eine zuvor beschriebene Bedeutung haben.

Vorzugsweise handelt es sich bei den Kationen der erfindungsgemäßen ionischen Flüssigkeit um Ammonium-, Phosphonium-, Imidazolium-, Pyridinium- oder Pyrrolidinium-Kationen.

Besonders bevorzugte lonische Flüssigkeiten sind Ammonium-, Phosphonium-, Imidazolium- oder Pyrrolidinium-Hydrogensulfate, - Alkylsulfate, -Alkylsulfonate, -Perfluoralkylsulfonate, -Phosphate, - Hydrogenphosphate, -Alkylphosphate, -Alkyl und Perfluoralkyl-phosphinate, -Alkyl und Perfluoroalkyl-phosphonate oder -Perfluoralkylcarboxylate.

Die erfindungsgemäße Reaktion lässt sich in folgendem Reaktionsschema zusammenfassen:

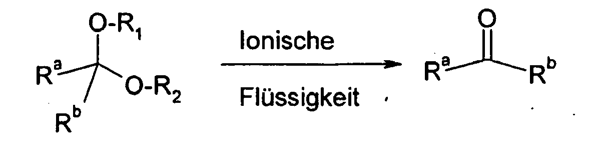

In einer weiteren bevorzugten Ausführungsform des erfindungsgemäßen Verfahrens enthält die mindestens eine lonische Flüssigkeit zusätzlich mindestens eine Säure, vorzugsweise eine zum Anion A⁻ korrespondierende Säure. Generell ist jede Säure für die Mischung mit der lonischen Flüssigkeit geeignet. Beispiele für bevorzugte Mischungen, die sich als besonders geeignet in den erfindungsgemäßen Verfahren erweisen, sind z.B. Mischungen aus lonischen Flüssigkeiten mit [HSO₄]⁻-Anionen und H₂SO₄. Alternative Beispiele sind Mischungen aus lonischen Flüssigkeiten mit [CF₃SO₃]⁻-Anionen und CF₃SO₃H oder Mischungen aus lonischen Flüssigkeiten mit [CF₃C(O)O]⁻-Anionen und CF₃C(O)OH. Die genannten Mischungen sind dabei als beispielhaft anzusehen ohne eine Begrenzung der Möglichkeiten der vorliegenden Erfindung darzustellen.

Der Anteil der Säure in der lonischen Flüssigkeit kann 0 bis 90 Gew.-%, bezogen auf die Mischung, betragen, vorzugsweise liegt er im Bereich von 0 bis 50 Gew.-%.

Die Verfahrenstemperatur ist an sich nicht kritisch und beträgt üblicherweise 0°C bis 170°C, vorzugsweise 20°C bis 120°C.

Die genannten Mischungen aus lonischen Flüssigkeiten und mindestens einer Säure eignen sich in besonderer Weise in den erfindungsgemäßen Verfahren, da die Ketalspaltung oder Acetalspaltung schneller abläuft, als mit der lonischen Flüssigkeit alleine. Darüber hinaus hat sich gezeigt, dass sich insbesondere Mischungen aus lonischen Flüssigkeiten und zu dem Anion A⁻ der lonischen Flüssigkeit korrespondierenden Säuren, dadurch auszeichnen, dass die Säure in der Mischung gering flüchtig ist, das heißt in der Mischung in gleich bleibender Konzentration auch bei höheren Temperaturen, vorhanden ist. So erweist sich beispielsweise Trifluoressigsäure in der Mischung mit einer lonischen Flüssigkeit mit einem Trifluoracetat-Anion als praktisch nicht flüchtig und weist nur einen geringen Dampfdruck auf.

Besonders vorteilhaft wird das erfindungsgemäße Verfahren zur Synthese von arylsubstituierten Aldehyden oder Ketonen eingesetzt, die z.B. als mesogene Substanzen, Pharmawirkstoffe, Pflanzenschutzmittel, Polymere oder Vorstufen in der Feinchemie oder zur Herstellung entsprechender Ausgangsverbindungen Verwendung finden.

Es versteht sich für den Fachmann von selbst, dass in den genannten lonischen Flüssigkeiten oder Verbindungen der Formel (I) Substituenten wie beispielsweise H, N, O, Cl, F durch die entsprechenden Isotope ersetzt sein können.

Die folgenden Ausführungsbeispiele sollen die Erfindung erläutern, ohne sie zu begrenzen. Vor- und nachstehend bedeuten Prozentangaben Gewichtsprozent. Alle Temperaturen sind in Grad Celsius angegeben.

### Beispiele:

### Beispiel 1: Synthese von Cyclohex-1-on

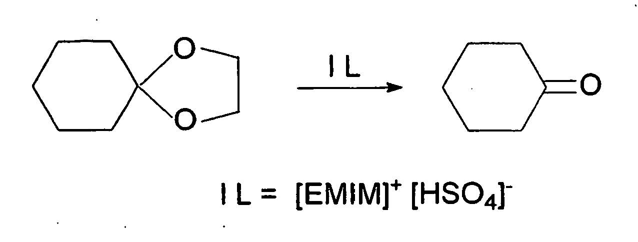

Zu 50 ml 1-Ethyl-3-Methyl-imidazoliumhydrogensulfat in einem 100 ml-Rundkolben werden 15.5 g (109.0 mmol) of Cyclohexanonethylenketal zugegeben und die Mischung wird bei 90-100°C für eine halbe Stunde gerührt. Danach werden die flüchtigen Bestandteile bei einem Druck von 35 mbar unter Verwendung einer Vigreuxkolonne (20 cm) innerhalb von 4 Stunden abdestilliert.. Es werden 5.58 g einer klaren und farblosen Flüssigkeit isoliert, die sich mit der Zeit entmischen. Die untere (2.14 g) der beiden Phasen besteht aus einer Lösung von reinem Cyclohexanon und Säure und wird wieder zurück zur ionischen Flüssigkeit gegeben. Die obere Phase (3.44 g) enthält Cyclohexanon, C₆H₁₀O (Reinheit 97 %). Zu der zurückbleibenden ionischen Flüssigkeit (Emulsion) werden weitere 22.4 g (157.5 mmol) Cyclohexanonethylenketal, C₈H₁₄O₂, zugegeben und erneut eine Stunde bei 90-100°C gerührt. Nach 2-stündiger Destillation bei einem Druck von 35 mbar wird eine klare und farblose Flüssigkeit (7.21 g) isoliert, die sich mit der Zeit entmischt. Die untere (3.00 g) der beiden Phasen besteht aus reinem Cyclohexanon und Säure und wird wieder zurück zur ionischen Flüssigkeit gegeben. Die obere Phase (3.44 g) enthält Cyclohexanon, C₆H₁₀O (Reinheit 96 %), das abgetrennt wird. Beim weiteren Destillieren im Vakuum (5 mbar) kann weiteres Destillat (12.2 g) von zwei Substanzen: Cyclohexanonethylenketal, C₈H₁₄O₂ (50 mol. %) und Cyclohexanon, C₆H₁₀O (50 mol. %) erhalten werden. Cyclohexanon kann aus diesem Destillat in einem weiteren Schritt isoliert werden. Anschließend muss die Schwefelsäure zum Reaktionsgemisch zugegeben werden um weitere Umsetzungen durchzuführen.
Der genannte Vorgang kann mehrfach wiederholt werden, ohne die ionische Flüssigkeit zu wechseln.

Das Produkt, Cyclohexanon, wird mittels NMR-Spektroskopie charakterisiert.

### Cyclohexanon:

¹H-NMR (Referenz: TMS; Lösungsmittel: CD₃CN), ppm: 1.64 m (2H); 1.79 m (4H); 2.26 m (4H).

¹³C {¹H} NMR (Referenz: TMS; Lösungsmittel: CD₃CN), ppm: 25.9 s (CH₂); 28.1 s (2CH₂); 42.7 s (2CH₂); 211.0 s (C=O)

### Beispiel 2: Synthese von Aceton

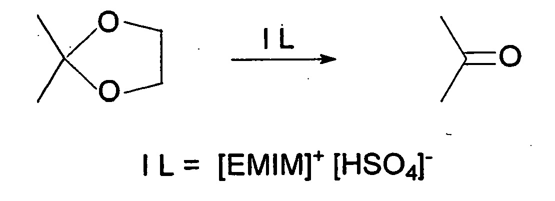

Zu 3 ml 1-Ethyl-3-Methyl-imidazoliumhydrogensulfat in einem 25 ml-Rundkolben werden 3.54 g (34.7 mmol) 2,2-Dimethyl-1,3-dioxolan zugegeben und die Mischung (Emulsion) wird bei 70°C für sechs Stunden gerührt. Danach werden alle flüchtigen Bestandteile bei 10⁻² mbar und 85-90°C abgepumpt und in einer Kühlfalle bei -196°C eingefroren. Es werden nach Aufarbeitung 1.92 g Aceton mit einer Reinheit von 92% erhalten. Die Ausbeute an Aceton beträgt 88 %.

### Aceton:

¹H NMR (Referenz: TMS; Lösungsmittel: CD₃CN), ppm: 2.09 s (6H)

¹³C{¹H} NMR (Referenz: TMS; Lösungsmittel: CD₃CN), ppm: 31.1 s (CH₃), 206.9 s (CO).

Der genannte Vorgang kann mehrfach wiederholt werden, ohne die ionische Flüssigkeit zu wechseln.

Vergleichbare Ergebnisse werden erhalten bei Einsatz einer Mischung aus EMIM Hydrogensulfat und 0.5 Mol H₂SO₄ (bezogen auf die Ausgangsverbindung).

### Beispiel 3: Synthese von Acetaldehyd

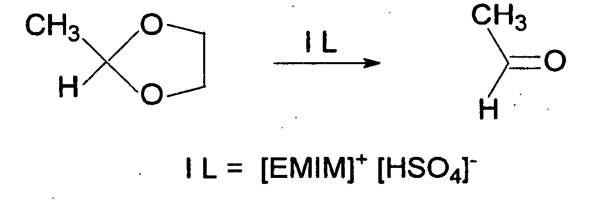

Zu 3 ml 1-Ethyl-3-Methyl-imidazoliumhydrogensulfat in einem 25 ml-Rundkolben werden 1.49 g (16.9 mmol) 2-Methyl-1,3-dioxolan zugegeben und die Mischung (Emulsion) wird bei 64°C für sechs Stunden gerührt. Danach werden alle flüchtigen Bestandteile bei 10⁻² mbar und 85 - 90°C abgepumpt und in einer Kühlfalle bei -196°C eingefroren. Nach Erwärmung auf Raumtemperatur werden 0.84 g einer farblosen Flüssigkeit erhalten. Gemäß der ¹H NMR-Spektroskopie enthält das Destillat zwei Verbindungen, nämlich 2-Methyl-1,3-dioxolan (ca. 13 mol. %) und Acetaldehyd (ca. 87 mol. %). Das Produkt, Acetaldehyd, kann durch fraktionierende Destillation aufgereinigt werden.

### Acetaldehyd:

¹H NMR (Referenz: TMS; Lösungsmittel: CD₃CN), ppm: 2.10 d (CH₃); 9.68 q (CHO); ³*J*_{HH} = 2.9 Hz.

¹³C{¹H} NMR (Referenz: TMS; Lösungsmittel: CD₃CN), ppm: 31.1 s (CH₃); 201.1 s (CHO).

Der genannte Vorgang kann mehrfach wiederholt werden, ohne die ionische Flüssigkeit zu wechseln.

Vergleichbare Ergebnisse werden erhalten bei Einsatz einer Mischung aus EMIM Hydrogensulfat und 0.5 Mol H₂SO₄ (bezogen auf die Ausgangsverbindung), wobei zusätzlich das Produkt der Aldolreaktion, hauptsächlich 3-Methylacrolein in der Reaktionsmischung gefunden wird.

### Beispiel 4: Synthese von Aceton

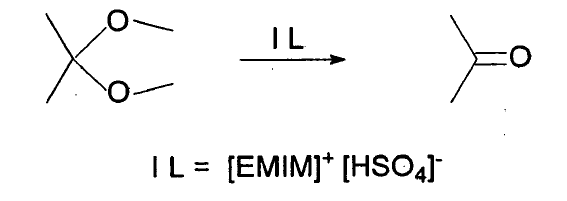

Zu 6 ml 1-Ethyl-3-Methyl-imidazoliumhydrogensulfat in einem 25 ml-Rundkolben werden 3.81 g (36.5 mmol) 2,2-Dimethoxypropan zugegeben und die Mischung (Emulsion) wird bei 55 - 60 °C für sechs Stunden gerührt. Danach werden alle flüchtigen Bestandteile bei 1 mbar und 55 - 60°C abdestilliert und in einer Kühlfalle bei -196°C eingefroren. Nach Erwärmung auf Raumtemperatur werden 3.38 g einer farblosen Flüssigkeit erhalten. Gemäß der ¹H NMR-Spektroskopie enthält das Destillat zwei Verbindungen, Aceton (ca. 62 mol. %) und Dimethylether (ca. 38 mol. %). Das Produkt, Aceton, wird durch fraktionierende Destillation aufgereinigt und mittels NMR-Spektroskopie charakterisiert.

### Aceton:

¹H NMR (Referenz: TMS; Lösungsmittel: CD₃CN), ppm: 2.04 s

¹³C {¹H} NMR (Referenz: TMS; Lösungsmittel: CD₃CN), ppm: 30.8 s (CH₃), 206.6 s (CO).

### Dimethylether:

¹H NMR (Referenz: TMS; Lösungsmittel: CD₃CN), ppm: 3.30 s.

¹³C {¹H} NMR (Referenz: TMS; Lösungsmittel: CD₃CN), ppm: 49.8 s (CH₃).

Der genannte Vorgang kann mehrfach wiederholt werden, ohne die ionische Flüssigkeit zu wechseln.

## Patentansprüche

1. Verfahren zur Spaltung von Dialkoxyalkanen der allgemeinen Formel (I) unter Erhalt von Aldehyden oder Ketonen 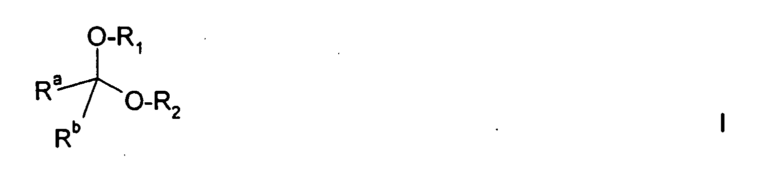 wobei
R^{a} und R^{b} unabhängig voneinander einen ggf. substituierten aliphatischen oder aromatischen Rest, der ein oder mehrere Heteroatome aufweisen kann, bedeuten, wobei einer der Reste R^{a} oder R^{b} auch H bedeuten kann, und wobei die beiden Reste R^{a} und R^{b} miteinander verbunden sein können, und wobei
R₁ und R₂ unabhängig voneinander einen ggf. substituierten gesättigten oder ungesättigten aliphatischen Rest oder aromatischen Rest, der ein oder mehrere Heteroatome aufweisen kann, bedeuten und wobei die beiden Reste R₁ und R₂ miteinander verbunden sein können, **dadurch gekennzeichnet, dass** die Spaltung in Anwesenheit mindestens einer lonischen Flüssigkeit der allgemeinen Formel K⁺A⁻ erfolgt.

2. Verfahren gemäß Anspruch 1, **dadurch gekennzeichnet, dass** das Anion A- der lonischen Flüssigkeit ausgewählt ist aus der Gruppe [HSO₄]⁻, [SO₄]²⁻, [NO₃]⁻, [BF₄]⁻, [(R_{F})BF₃]⁻, [(R_{F})₂BF₂]⁻, [(R_{F})₃BF]⁻, [(R_{F})₄B]⁻, [B(CN)₄]⁻, [PO₄]³⁻, [HPO₄]²⁻, [H₂PO₄]⁻, [Alkyl-OPO₃]²⁻, [(Alkyl-O)₂PO₂]⁻, [Alkyl-PO₃]²⁻, [R_{F}PO₃]²⁻, [(Alkyl)₂PO₂]⁻, [(R_{F})₂PO₂]⁻, [R_{F}SO₃]⁻, [HOSO₂(CF₂)ₙSO₂O]⁻, [OSO₂(CF₂)ₙSO₂O]²⁻, [Alkyl-SO₃]⁻, [HOSO₂(CH₂)ₙSO₂O]⁻, [OSO₂(CH₂)ₙSO₂O]²⁻, [Alkyl-OSO₃]⁻, [Alkyl-C(O)O]⁻, [HO(O)C(CH₂)ₙC(O)O]⁻, [R_{F}C(O)O]⁻, [HO(O)C(CF₂)ₙC(O)O]⁻, [O(O)C(CF₂)ₙC(O)O]²⁻, [(R_{F}SO₂)₂N]⁻, [(FSO₂)₂N]⁻, [((R_{F})₂P(O))₂N]⁻, [(R_{F}SO₂)₃C]⁻, [(FSO₂)₃C]⁻, Cl⁻ und/oder Br⁻, wobei n = 1 bis 8 bedeutet und R_{F} die Bedeutung fluoriertes Alkyl
(CₘF₂ₘ₋ₓ₊₁Hₓ)
mit m = 1- 12 und x = 0- 7 hat, wobei für m = 1 x = 0 bis 2 sein soll, und/oder fluoriertes Aryl oder Alkyl-Aryl hat.

3. Verfahren gemäß Anspruch 1 oder 2, **dadurch gekennzeichnet, dass** die Kationen K⁺ der lonischen Flüssigkeit ausgewählt sind aus der Gruppe umfassend Ammonium-, Phosphonium, Thiouronium-, Guanidiniumkationen oder heterocyclische Kationen.

4. Verfahren gemäß einem oder mehreren der Ansprüche 1 bis 3, **dadurch gekennzeichnet, dass** die mindestens eine lonische Flüssigkeit zusätzlich eine zum Anion A⁻ korrespondierende Säure enthält.

5. Verfahren gemäß Anspruch 4, dass der Anteil der Säure in der lonischen Flüssigkeit 0 bis 90 Gew.-%, bezogen auf die Mischung beträgt.

6. Verfahren gemäß einem oder mehreren der Ansprüche 1 bis 5, **dadurch gekennzeichnet, dass** die Verfahrenstemperatur 0°C bis 170°C beträgt.

7. Verfahren gemäß einem oder mehreren der Ansprüche 1 bis 6, **dadurch gekennzeichnet, dass** R^{a} eine Bedeutung der Formel la aufweist 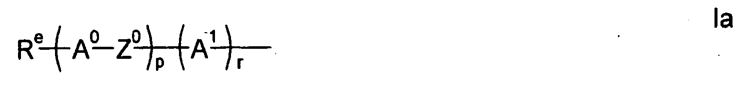 worin
R^{e} einen unsubstituierten, einen einfach oder mehrfach durch CN und/oder Halogen substituierten Alkylrest mit 1 bis 15 C-Atomen, wobei in diesen Resten auch eine oder mehrere CH₂-Gruppen durch -O-, -S-, -CH=CH-, -C≡C-, -OC-O- und/oder -O-CO-, und/oder auch eine oder mehrere CH-Gruppen durch N oder P so ersetzt sein können, dass zwei O-Atome nicht direkt miteinander verknüpft sind, oder, falls r und/oder p verschieden von 0 sind, auch H, Halogen, CN, SF₅ oder NCS,
A⁰, A¹ jeweils unabhängig voneinander
a) einen 1,4-Cyclohexenylen- oder 1,4-Cyclohexylenrest, worin eine oder zwei nicht benachbarte CH₂-Gruppen durch -O- oder -S- ersetzt sein können,
b) einen 1,4-Phenylenrest, worin eine oder zwei CH-Gruppen durch N ersetzt sein können,
c) einen Rest aus der Gruppe Piperidin-1,4-diyl, 1,4-Bicyclo[2,2,2]octylen, Phenanthren-2,7-diyl, Naphthalin-2,6-diyl, Decahydronaphthalin-2,6-diyl, und 1,2,3,4-Tetrahydronaphthalin-2,6-diyl,
d) einen bivalenten Rest aus der Gruppe Furan, Pyrrol, Thiophen, Pyrazol, Imidazol, 1,2-Oxazol, 1,3-Oxazol, Thiazol, Pyridin, Pyridazin, Pyrimidin, Pyrazin, 2H-Pyran, 4H-Pyran, Purin, Pteridin, 1 H-Azepin, 3H-1,4-Diazepin, Indol, Benzofuran, Benzothiophen, Chinolin, Isochinolin, Phenazin, Phenoxazin, Phenothiazin und 1,4-Benzodiazepin,
wobei die Reste a), b), c) und d) ein oder mehrfach durch R^{e}, insbesondere durch Halogen und/oder CN, substituiert sein können,
Z⁰ -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- oder eine Einfachbindung,
p 0, 1, 2 oder 3 und
r 0, 1 oder 2 bedeutet.

8. Verfahren nach einem oder mehreren der Ansprüche 1 bis 7, **dadurch gekennzeichnet, dass** der Rest R^{a} eine Bedeutung gemäß der Formel la nach Anspruch 7 besitzt und der Rest R^{b} H bedeutet.

## Claims

1. Process for the cleavage of dialkoxyalkanes of the general formula (I) to give aldehydes or ketones 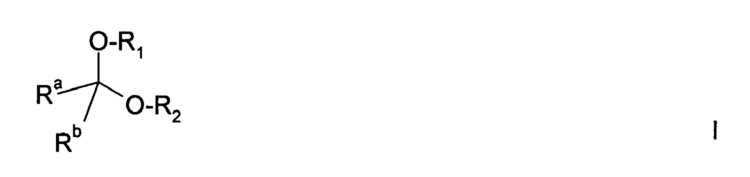 where
R^{a} and R^{b}, independently of one another, denote an optionally substituted aliphatic or aromatic radical, which may have one or more heteroatoms, where one of the radicals R^{a} or R^{b} may also denote H, and where the two radicals R^{a} and R^{b} may be connected to one another, and where
R₁ and R₂, independently of one another, denote an optionally substituted saturated or unsaturated aliphatic or aromatic radical, which may have one or more heteroatoms, and where the two radicals R₁ and R₂ may be connected to one another, **characterised in that** the cleavage is carried out in the presence of at least one ionic liquid of the general formula K⁺A⁻.

2. Process according to Claim 1, **characterised in that** the anion A⁻ of the ionic liquid is selected from the group [HSO₄]⁻, [SO₄]²⁻, [NO₃]⁻, [BF₄]⁻, [(R_{F})BF₃]⁻, [(R_{F})₂BF₂]⁻, [(RF)₃BF]⁻, [(R_{F})₄B]⁻, [B(CN)₄]⁻, [PO₄]³⁻, [HPO₄]²⁻, [H₂PO₄]⁻, [alkyl-OPO₃]²⁻, [(alkyl-O)₂PO₂]⁻, [alkyl-PO₃]²⁻, [R_{F}PO₃]²⁻, [(alkyl)₂PO₂]⁻, [(R_{F})₂PO₂]⁻, [R_{F}SO₃]⁻, [HOSO₂(CF₂)ₙSO₂O]⁻, [OSO₂(CF₂)ₙSO₂O]²⁻, [alkyl-SO₃]⁻, [HOSO₂(CH₂)ₙSO₂O]⁻, [OSO₂(CH₂)ₙSO₂O]²⁻, [alkyl-OSO₃]⁻, [alkyl-C(O)O]⁻, [HO(O)C(CH₂)ₙC(O)O]⁻, [R_{F}C(O)O]⁻, [HO(O)C(CF₂)ₙC(O)O]⁻, [O(O)C(CF₂)ₙC(O)O]²⁻, [(R_{F}SO₂)₂N]⁻, ((FSO₂)₂N]⁻, [((R_{F})₂P(O))₂N]⁻, [(R_{F}SO₂)₃C]⁻, [(FSO₂)₃C]⁻, Cl⁻ and/or Br⁻, where n = 1 to 8, and R_{F} has the meaning of fluorinated alkyl
(CₘF₂ₘ₋ₓ₊₁Hₓ)
where m = 1-12 and x = 0-7, where, for m = 1, x must be = 0 to 2, and/or fluorinated aryl or alkylaryl.

3. Process according to Claim 1 or 2, **characterised in that** the cations K⁺ of the ionic liquid are selected from the group consisting of ammonium, phosphonium, thiouronium, guanidinium and heterocyclic cations.

4. Process according to one or more of Claims 1 to 3, **characterised in that** the at least one ionic liquid additionally comprises an acid corresponding to the anion A⁻.

5. Process according to Claim 4, **characterised in that** the proportion of acid in the ionic liquid is 0 to 90% by weight, based on the mixture.

6. Process according to one or more of Claims 1 to 5, **characterised in that** the process temperature is 0°C to 170°C.

7. Process according to one or more of Claims 1 to 6, **characterised in that** R^{a} has a meaning of the formula la 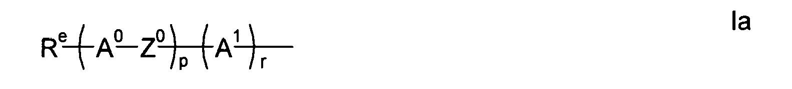 in which
R^{e} denotes an alkyl radical having 1 to 15 C atoms which is unsubstituted, mono- or polysubstituted by CN and/or halogen, where, in addition, one or more CH₂ groups in these radicals may be replaced by -O-, -S-, -CH=CH-, -C≡C-, -OC-O-and/or -O-CO-, and/or, in addition, one or more CH groups may be replaced by N or P in such a way that two O atoms are not linked directly to one another, or, if r and/or p are different from 0, also denotes H, halogen, CN, SF₅ or NCS,
A⁰, A¹ each, independently of one another, denote
a) a 1,4-cyclohexenylene or 1,4-cyclohexylene radical, in which one or two non-adjacent CH₂ groups may be replaced by -0- or -S-,
b) a 1,4-phenylene radical, in which one or two CH groups may be replaced by N,
c) a radical from the group piperidine-1,4-diyl, 1,4-bicyclo-[2.2.2]octylene, phenanthrene-2,7-diyl, naphthalene-2,6-diyl, decahydronaphthalene-2,6-diyl and 1,2,3,4-tetra-hydronaphthalene-2,6-diyl,
d) a divalent radical from the group furan, pyrrole, thiophene, pyrazole, imidazole, 1,2-oxazole, 1,3-oxazole, thiazole, pyridine, pyridazine, pyrimidine, pyrazine, 2H-pyran, 4H-pyran, purine, pteridine, 1H-azepine, 3H-1,4-diazepine, indole, benzofuran, benzothiophene, quinoline, isoquinoline, phenazine, phenoxazine, phenothiazine and 1,4-benzodiazepine,
where the radicals a), b), c) and d) may be mono- or poly-substituted by R^{e}, in particular by halogen and/or CN,
Z⁰ denotes -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- or a single bond,
p denotes 0, 1, 2 or 3, and
r denotes 0, 1 or 2.

8. Process according to one or more of Claims 1 to 7, **characterised in that** the radical R^{a} has a meaning in accordance with formula la according to Claim 7, and the radical R^{b} denotes H.

## Revendications

1. Procédé de clivage de dialcoxyalkanes de formule générale (I) pour donner des aldéhydes ou des cétones 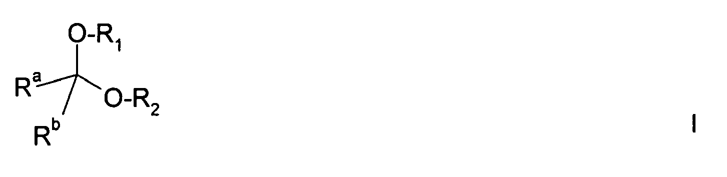 dans laquelle
R^{a} et R^{b}, indépendamment l'un de l'autre, désignent un radical aliphatique ou aromatique éventuellement substitué, pouvant posséder un ou plusieurs hétéroatomes, où l'un des radicaux R^{a} ou R^{b} peut également désigner H, et où les deux radicaux R^{a} et R^{b} peut être reliés l'un à l'autre, et où
R₁ et R₂, indépendamment l'un de l'autre, désignent un radical aliphatique ou aromatique, saturé ou non saturé, éventuellement substitué, pouvant posséder un ou plusieurs hétéroatomes, et où les deux radicaux R₁ et R₂ peut être reliés l'un à l'autre, **caractérisé en ce que** le clivage est effectué en présence d'au moins un liquide ionique de formule générale K⁺A⁻.

2. Procédé selon la revendication 1, **caractérisé en ce que** l'anion A⁻ du liquide ionique est choisi parmi le groupe constitué par [HSO₄]⁻, [SO₄]²⁻, [NO₃]⁻, [BF₄]⁻, [(R_{F})BF₃]⁻. [(R_{F})₂BF₂]⁻, [(R_{F})₃BF]⁻, [(R_{F})₄B]⁻, [B(CN)₄]⁻, [PO₄]³⁻, [HPO₄]²⁻, [H₂PO₄]⁻, [alkyl-OPO₃]²⁻, [(alkyl-O)₂PO₂]⁻, [alkyl-PO₃]²⁻, [R_{F}PO₃]²⁻, [(alkyl)₂PO₂]⁻, [(R_{F})₂PO₂]⁻, [R_{F}SO₃]⁻ [HOSO₂(CF₂)ₙSO₂O]⁻, [OSO₂(CF₂)ₙSO₂O]²⁻, [alkyl-SO₃]⁻, [HOSO₂(CH₂)ₙSO₂O]⁻, [OSO₂(CH₂)ₙSO₂O]²⁻, [alkyl-OSO₃]⁻, [alkyl-C(O)O]⁻, [HO(O)C(CH₂)ₙC(O)O]⁻, [R_{F}C(O)O]⁻, [HO(O)C(CF₂)ₙC(O)O]⁻, [O(O)C(CF₂)ₙC(O)O]²⁻, [(R_{F}SO₂)₂N]⁻, [(FSO₂)₂N]⁻, [((R_{F})₂P(O))₂N]⁻, [(R_{F}SO₂)₃C]⁻, [(FSO₂)₃C]⁻, Cl⁻ et/ou Br⁻, où n = 1 à 8, et R_{F} signifie alkyle fluoré
(CₘF₂ₘ₋ₓ₊₁Hₓ)
où m = 1-12 et x = 0-7, où, pour m = 1, x doit être = 0 à 2, et/ou aryle ou alkylaryle fluoré.

3. Procédé selon la revendication 1 ou 2, **caractérisé en ce que** les cations K⁺ du liquide ionique sont choisis parmi le groupe constitué par les cations ammonium, phosphonium, thiouronium, guanidinium et hétérocycliques.

4. Procédé selon l'une ou plusieurs des revendications 1 à 3, **caractérisé en ce que** le au moins un liquide ionique comprend en outre un acide correspondant à l'anion A⁻.

5. Procédé selon la revendication 4, **caractérisé en ce que** la proportion d'acide dans le liquide ionique va de 0 à 90% en poids, sur la base du mélange.

6. Procédé selon l'une ou plusieurs des revendications 1 à 5, **caractérisé en ce que** la température du procédé va de 0°C à 170°C.

7. Procédé selon l'une ou plusieurs des revendications 1 à 6, **caractérisé en ce que** R^{a} possède la signification de la formule la 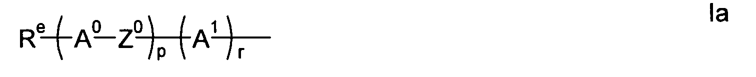 dans laquelle
R^{e} désigne un radical alkyle ayant de 1 à 15 atomes de C qui est non substitué, mono- ou polysubstitué par CN et/ou halogène, où, en outre, un ou plusieurs groupements CH₂ dans ces radicaux peuvent être remplacés par -O-, -S-, -CH=CH-, -C≡C-, -OC-O- et/ou -O-CO-, et/ou, en outre, un ou plusieurs groupements CH peuvent être remplacés par N ou P de telle sorte que deux atomes de O ne soient pas liés directement l'un à l'autre, ou, si r et/ou p sont différents de 0, désigne aussi H, halogène, CN, SF₅ ou NCS,
A⁰, A¹ chacun, indépendamment l'un de l'autre, désigne
a) un radical 1,4-cyclohexénylène ou 1,4-cyclohexylène, dans lequel un ou deux groupements CH₂ non adjacents peuvent être remplacés par -O- ou -S-,
b) un radical 1,4-phénylène, dans lequel un ou deux groupements CH peuvent être remplacés par N,
c) un radical issu du groupe constitué par pipéridine-1,4-diyle, 1,4-bicyclo[2.2.2]octylène, phénanthrène-2,7-diyle, naphtalène-2,6-diyle, décahydronaphtalène-2,6-diyle et 1,2,3,4-tétrahydronaphtalène-2,6-diyle,
d) un radical divalent issu du groupe constitué par furane, pyrrole, thiophène, pyrazole, imidazole, 1,2-oxazole, 1,3-oxazole, thiazole, pyridine, pyridazine, pyrimidine, pyrazine, 2H-pyranne, 4H-pyranne, purine, ptéridine, 1 H-azépine, 3H-1,4-diazépine, indole, benzofurane, benzothiophène, quinoléine, isoquinoléine, phénazine, phénoxazine, phénothiazine et 1,4-benzodiazépine, où les radicaux a), b), c) et d) peuvent être mono- ou poly-substitués par R^{e}, en particulier par halogène et/ou CN,
Z⁰ désigne -CO-O-, -O-CO-, -CF₂O-, -OCF₂-, -CH₂O-, -OCH₂-, -CH₂CH₂-, -(CH₂)₄-, -C₂F₄-, -CH₂CF₂-, -CF₂CH₂-, -CF=CF-, -CH=CH-, -C≡C- ou une liaison simple,
p désigne 0, 1, 2 ou 3, et
r désigne 0, 1 ou 2.

8. Procédé selon l'une ou plusieurs des revendications 1 à 7, **caractérisé en ce que** le radical R^{a} possède une signification conforme à la formule la selon la revendication 7, et le radical R^{b} désigne H.
